# EUROPEAN PATENT APPLICATION

(11) **EP 1 231 272 A2**
(43) Date of publication of application: **14.08.2002**
(21) Application number: 02380019.6
(22) Date of filing: 30.01.2002
(51) Int. Cl.: C12N 15/59, C12N 9/64, C12N 15/80, C12P 21/02, C12N 1/15

(54) **Procedure for obtaining bovine chymosin (rennin), curd**

(30) Priority: 08.02.2001 ES 200100286
(71) Applicant: Laboratorios Ovejero S.A., 24192 Leon (ES)
(72) Inventor: Elena, Cardoza Rosa, Dr., 24192 Leon (ES); Gutierrez Martin, Santiago, Dr., 24192 Leon (ES); Moralejo Lorenzo, Francisco Jos-, Dr., 24192 Leon (ES); Casqueiro Blanco, Francisco Javier, Dr., 24192 Leon (ES); Martin Martin, Juan Francisco, Dr., 24192 Leon (ES)
(74) Representative: Gil-Vega, Victor

(57) **Abstract**

A procedure for obtaining curd (bovine rennin) by the expression, not of the sequencing of its natural gene, but of an artificial gene, synthetic and optimised following certain rules of use of triplets in DNA, is described. Preferably, this expression is made with filamentous fungi, especially with GRAS status, and particularly Aspergillus niger, awamori variant. The synthesis of optimised genes for filamentous fungi, carried out here for the first time for chymosin, allows us to obtain high levels of expression, which means that the procedure is useful for the industrial production of this valuable protein. Chymosin is obtained extra-cellularly, using a plasmid with a fungal secretion signal, thus allowing for its purification by the supernatants from the growth of this fungus, for use in the food industry.

## Description

This invention, based on genetic engineering or recombinant DNA engineering, refers to a procedure for obtaining bovine chymosin from new DNA sequences that have been optimised for expression in filamentous fungi and which encode for this protein, and to the use of these sequences in the transformation of filamentous fungi for chymosin production.

### STATE OF THE ART

Chymosin, or rennin, is an enzyme found in the fourth stomach of calves (abomasum). It is an protease aspartyl (EC 3.4.4.15) and performs a limited proteolysis of the k-casein in milk, resulting in its clotting. This occurs during the first six weeks of the calf's lactancy, and is later replaced by pepsin A. Chymosin is formed in the calf's cells as a precursor of preprochymosin, a polypeptide with 16 amino-acids, from which a peptide of 16 amino-acids is eliminated during the secretion of the cells to give rise to prochymosin, which has a molecular weight of 40.5 kDa, and is also inactive. Under the acid conditions of the stomach (pH 4.5), the elimination of a peptide with 42 amino-acids is produced from the amino-terminal extreme, giving rise to active chymosin (molecular weight 35.6 kDa) (Pedersen et al, 1979).

In spite of the value of this protein, its industrial production is costly because of the small number of calves slaughtered, and its availability, therefore, is limited.

There are many examples of chymosin production by genetic engineering in organisms such as Escherichia Coli (Emtage at al, 1983), Saccharomyces cerevisiae (Mellor et al, 1983), in filamentous fungi such as Aspergillus nidulans (Cullen et al, 1987), Trichoderma reesei (Pitts et al, 1991), Aspergillus awamori (Dunn-Coleman et al, 1991), Aspergillus oryzae (Tsuchiya et al, 1993, 1994). In all the previous examples the natural gene encoded for prochymosin was used and the sequence was obtained from the cDNA obtained from a calf's stomach.

(It is important to point out that the production levels obtained by the use of the cDNA that encodes for prochymosin have been satisfactory when expressed in filamentous fungi. However, this production is covered by other patents that prevent it from being used for production by Spanish companies.)

### EXPLANATION OF THE INVENTION

This invention solves the problem of the preparation of chymosin by expression in filamentous fungi, not using the natural gene (or the derived cDNA) as was described for the species of the Aspergillus family, but using an artificial gene, synthetic and optimised for expression in filamentous fungi. The procedure for obtaining an artificial gene optimised for filamentous fungi, carried out here for the first time in the case of chymosin, allows for high protein expressions, which means that the procedure can be used for industrial purposes.

A particular embodiment of this invention is that in which the filamentous fungi used are considered to be harmless, particularly those with GRAS (Generally Recognised as Safe) status. The preferred GRAS fungi are members of the Aspergillus family, particularly of the Aspergillus niger species, awamori variant.

This invention consists of the procedure for obtaining excreted or extra-cellularly produced chymosin, for which an adequate secretion signal has to be introduced into the plasmid.

The following abbreviations will be used, among others:
A = adenine
Amp = ampicillin
C = cytosine
DNA = deoxyribonucleic acid
Da = Dalton
G = guanine
GRAS = Generally recognised as safe
kDa = Kilodalton
pb = base pairs
PCR = Polymerase Chain Reaction
PEG = polyethylenglycol
rpm = revolutions per minute
SDS = dodecyl sodium sulphate
T = thymine
X-gal = 5-bromine-4-chlorine-3-indo-β-D-galactose

The amino-acids are referred to their standard three-letter abbreviations, in English. For plasmids we use the note published in each case.

### 1.- Design of the synthetic chy gene with the use of Aspergillus niger codons

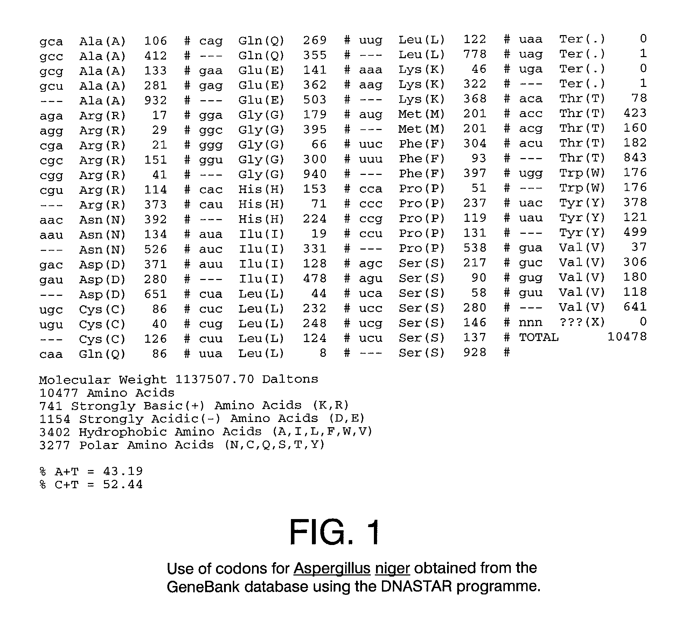

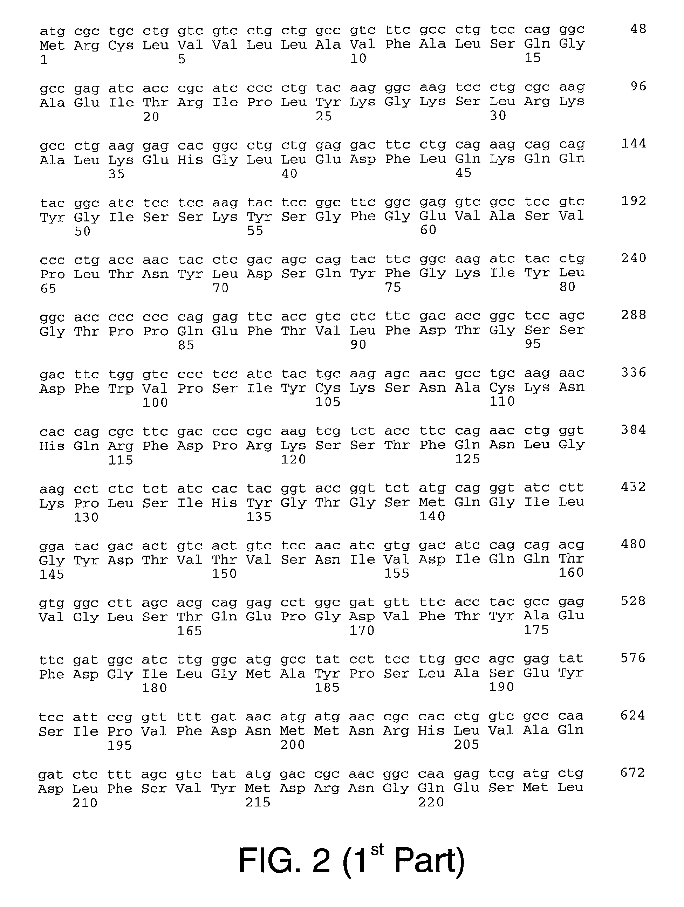

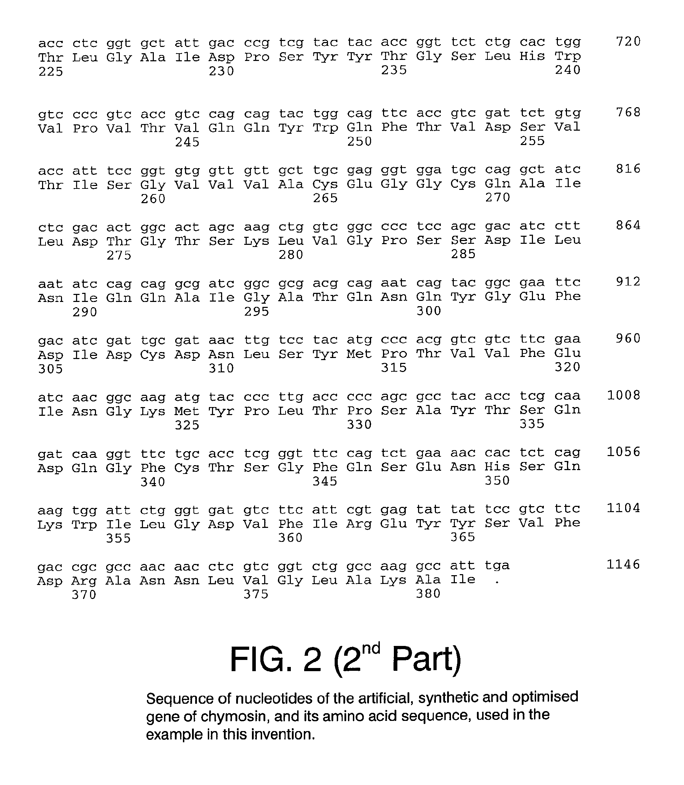

For the synthesis of the chy gene for the production of calf's chymosin in Aspergillus awamori, the first thing was to design a chy gene adapted to the use of codons from the closest possible species. In this case we decided to use Aspergillus niger codons. We obtained a bank of A. niger DNA sequences with open reading frameworks to complete a total of 31,434 pairs of encoding bases, which encoded for proteins with a total of 10,477 amino-acids. The table showing the use of codons (in Figure 1) allowed us to design a sequence of nucleotides with 1,146 pb which encoded for a protein identical to bovine chymosin (Figure 2) with a sequence of nucleotides that adapted perfectly to the use of Aspergillus awamori codons.

### 2.- Comparative analysis of the amino-acid sequences of synthetic chymosin from A. awamori with bovine chymosin

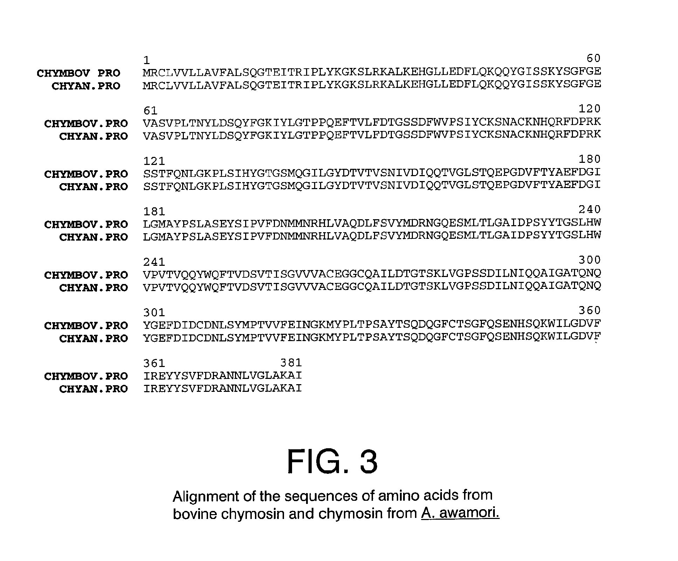

The comparative analysis of the amino-acid sequences and the hydrophobicity profiles, flexible regions and antigenic index, showed that both mature proteins were identical (Figure 3).

### 3.- Strategy used for the synthesis of the chy gene from A. awamori

### 3.1.- Synthesis of the first 475 pb

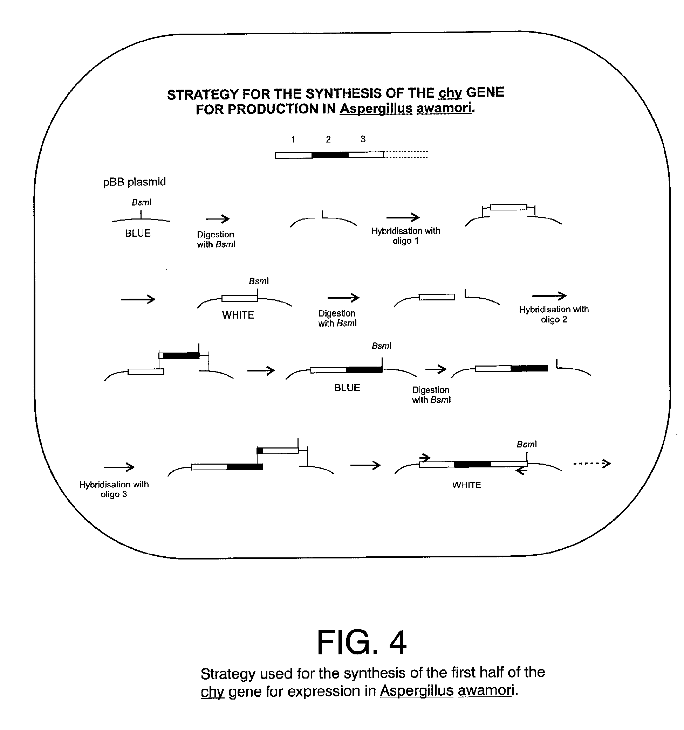

Once the nucleotide sequence was known, the strategy used was initially the one described by Hayden and Mandecki (1988), which is shown on Figure 4. This method consists of the use of a plasmid, in our case the pBB plasmid (built in our laboratory for this purpose) that contains a single *BsmI* restriction endonuclease cut site, and which also has in the framework of this cut site the gene that it encodes for a sub-unit of the β-galactosidase from E. coli, so that in the presence of the X-gal substrate in the media, the clones expressed by this enzyme are a blue colour and those that are not expressed by this enzyme are white. In addition to this plasmid, Escherichia coli stock with very active polymerase DNA is required, to easily repair large gaps in the DNA. In our case we used JM83 E. coli stock, which complies with the two previous conditions. Finally, the gene to be synthesised has to be divided into fragments between 20 and 50 bases each and oligonucleotides with the sequence of each of these fragments have to be synthesised. In the case of the A. awamori chy gene, it was divided into 20 similarly but not identically sized fragments.

The synthesis of the chy gene started with the digestion of the plasmid with *BsmI,* generating a linear plasmid with *BsmI* ends. This plasmid was mixed with the oligonucleotide that corresponded to the first fragment of the gene. It is important to emphasise that the oligonucleotide not only contains the sequence of the fragment to be lengthened, but it must contain at both ends sequences that are homologous with the ends of the plasmid, so that hybridisation takes place between them. This mix was de-naturalised by heat and then left to ring by incubation at room temperature, and finally introduced by transformation into the JM83 E. coli stock that was previously mentioned. Finally, the transformants were selected in an LB media with the ampicillin antibiotic and with X-gal to see the colour of the colonies. It is also important to emphasise that the oligonucleotides were designed alternating the colour resulting from the corresponding transformants in a media with X-gal, so that one oligonucleotide maintained the framework of the plasmid's β-galactosidase and the next one did not, and the clones were alternatively blue and white, thus simplifying the analysis of the clones obtained (Figure 4).

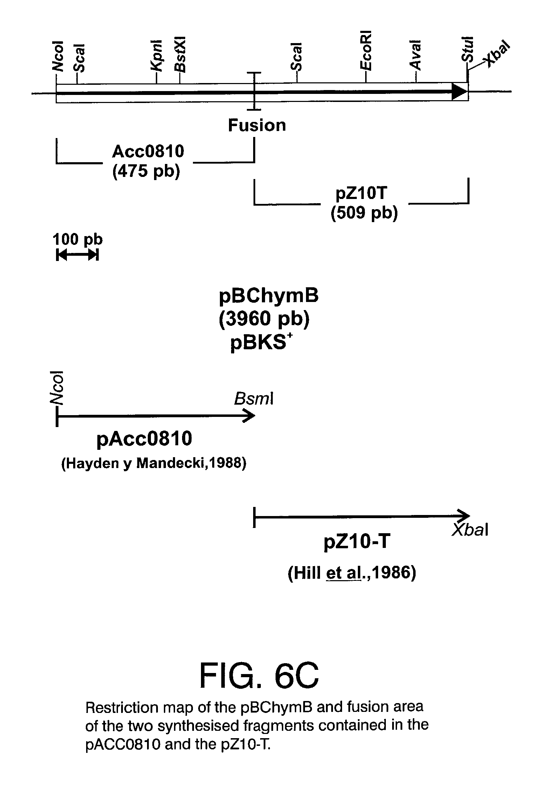

Using this method, it was possible to synthesis 475 pair bases. The plasmid that contains this fragment was called pAcc0810 (Figure 6C).

### 3.2.- Synthesis of the second half of the chy gene

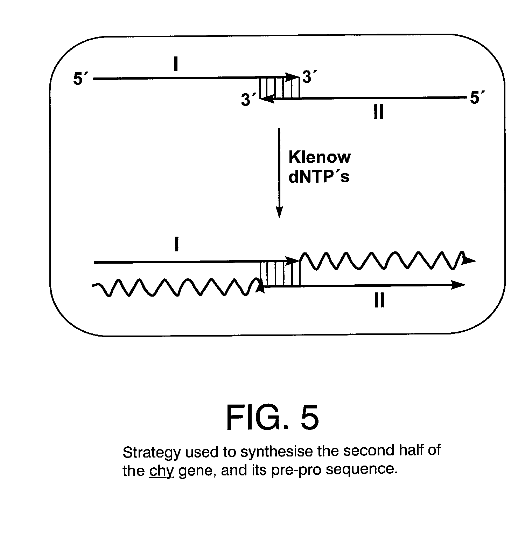

The other alternative for the synthesis of the second half of the chy gene that was used was the method described by Matteucci and Heyneker (1983) and by Hill et al. (1986), which consisted of using two large oligonucleotides that covered the area that had not yet been synthesised and with an overlapping area of at least 18 bases. This oligos are aligned and the uncompleted areas are filled with the Klenow fragment of the polymerase DNA. Once the sequence of both chains is completed, the resulting fragment is cloned in the relevant plasmid to complete the synthesis of the chy gene. A diagram of this method is shown on figure 5.

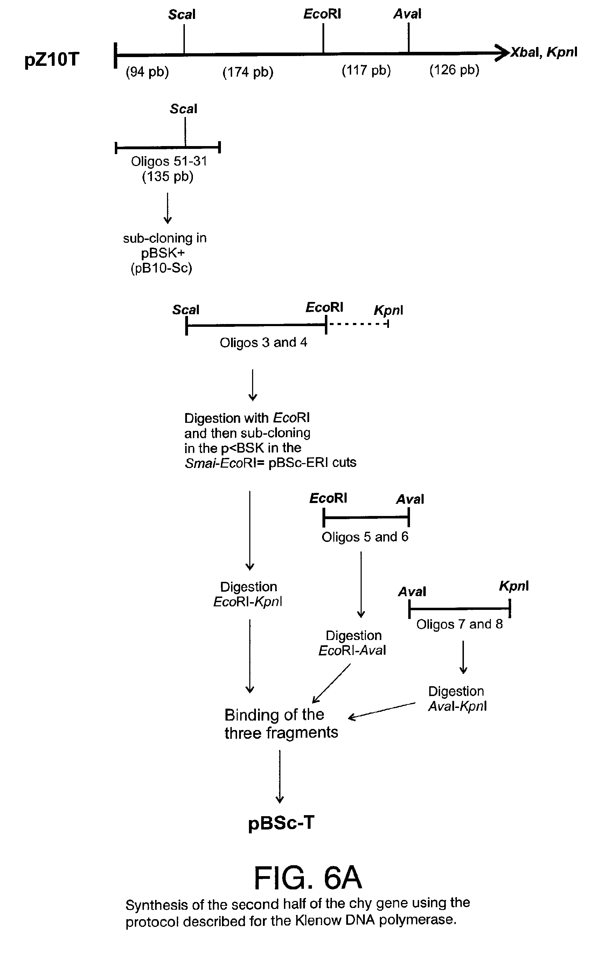

To complete the second half of the chy gene, it was necessary to design 10 oligonucleotides of different sizes that covered the area that had not yet been synthesised. The first oligonucleotides designed were called 51 and 31 and they corresponded to a fragment of the 135 pb gene (position 475-610 of mature chymosin, 649-784 on figure 2). Once the synthesis protocol described in figure 5 was completed, this fragment was sub-cloned in the pBluescript KS+ plasmid on the *EcoRV* cut site. Five positive clones were analysed by sequence in the two orientations using Perkin Elmer ABIPRISM equipment. Of the clones analysed, 85% had a satisfactory result, meaning that the synthesis had been efficiently carried out by this protocol. The resulting plasmid was called pB10-Sc (Figure 6A). 6 more oligonucleotides were then designed, making the most of the single cut sites of the nucleotidic gene sequence. Oligonucleotides 3 and 4 were therefore designed, with *ScaI* and *EcoRI* cut sites on the ends of each, respectively, to give rise to a 174 pb fragment, oligonucleotides 5 and 6 with *EcoRI* and *AvaI* ends, respectively (117 pb) and oligonucleotides 7 and 8 with *AvaI* ends on end 3' of the fragment (oligo 7) and with cut sites *StuI, XbaI* and *KpnI* on end 3' (oligo 8), respectively, to facilitate further cloning experiments. The sub-cloning of all these fragments is described on Figure 6A (at each stage of the different synthesis and sub-cloning experiments, we have performed the sequencing of these fragments in both directions).

The synthesised fragment with oligos 3 and 4 was sub-cloned in pBluescript SK+ plasmid at cut sites *SmaI* -EcoRI,* (in this case this fragment was not digested with the *ScaI* enzyme because there is a cut site on the vector used that is located in the sequence that encodes for the β-lactamase gene which provides resistance to ampicillin). The resulting plasmid was called pBSc-ERI. It was later digested with the *EcoRI* and *KpnI* enzymes and set to bind with the fragments synthesised from the 5-6 and 7-8 oligonucleotides previously digested with the *EcoRI-AvaI* and *AvaI-Kpni* enzymes, respectively. The resulting construction was called pBSc-T (Figure 6A).

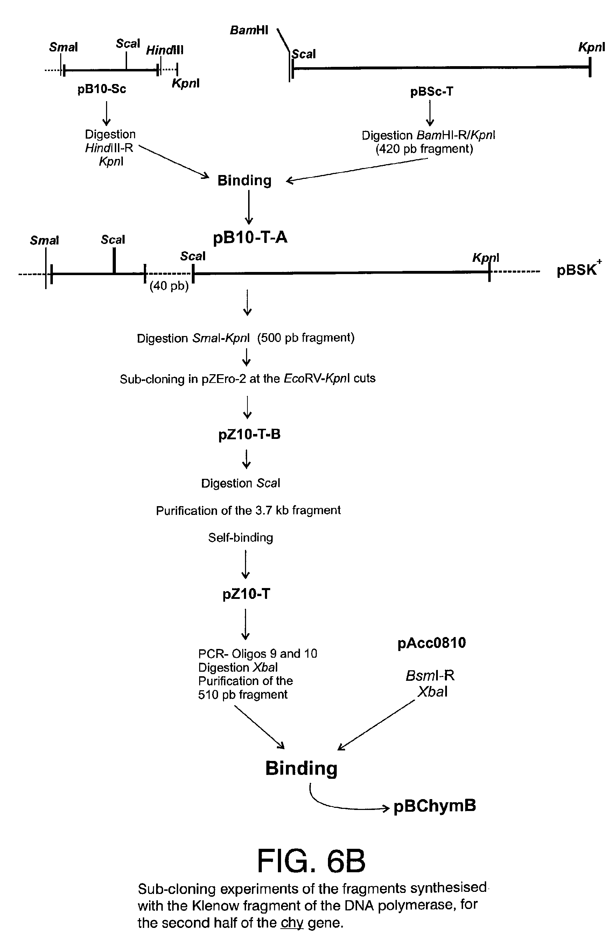

The pBSc-T plasmid was digested with the *BamHI* enzyme, and later the resulting 5' end was filled with the Klenow fragment and then digested with the *KpnI* enzyme. As a result of this treatment, a 420 pb fragment was released, which was recovered through an agarose gel. This fragment contained 417 pb corresponding to the chy gene. The resulting fragment was sub-cloned in the pB10-Sc plasmid, previously treated with *HindIII,* the Klenow fragment and the *KpnI* enzyme, giving rise to the pB10-T-A plasmid. This plasmid contained the synthesised fragment that corresponded to the first half of the chy gene and the three later fragments synthesised from the pBSc-T plasmid in the same direction with an intermediate 40 pb area corresponding to the vector used (Figure 6B). The pB10-T-A was digested with the *SmaI-KpnI* enzymes, obtaining a 500 pb fragment that was sub-cloned in the pZEro-2 plasmid (Invitrogen, Netherlands) at cut sites *EcoRV* and *KpnI,* giving rise to the plasmid pZ10-T-B. The plasmid pZ10-T-B was digested by the *Scal* enzyme, this fragment (3.7 pb) was purified and left to self-bind, giving rise to the plasmid pZ10-T, which finally contained the second half of the complete chy gene (Figure 6B).

Finally, Figure 6B shows the experiment that was conducted for the fusion of the two fragments of the chy gene. The plasmid pAcc0810 was first digested with the *BsmI* enzyme, later treated with the Klenow fragment and finally digested with the *XbaI* enzyme. On the other hand, from the plasmid pZ10-T and oligos 9 and 10, the 510 pb fragment corresponding to the second half of the chy gene was amplified by PCR using the *Pfu* polymerase (Stratagene, CA). This fragment was digested with the *XbaI* enzyme and bound to the linearised plasmid pAcc0810 as previously described, which gave rise to the plasmid pBChymB (3960 pb) that contained the gene that encodes for complete mature chymosin. Figure 6C shows a diagram of the pBChymB (984 pb) restriction map, identifying the place of fusion of the two fragments synthesised by the two methods used. This construction completed the synthesis of the sequence that corresponds to mature chymosin.

### 4.- Synthesis of the DNA fragment that corresponds to the pre-pro sequence of chymosin

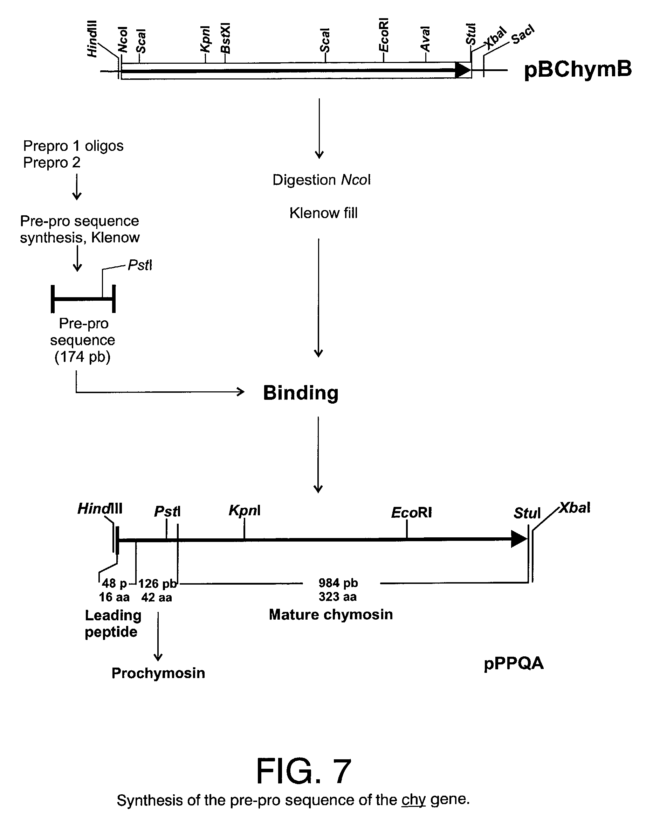

To synthesise the pre-pro sequence of chymosin, we designed oligos prepro-1 and prepro-2. The synthesis protocol followed was as described in figure 5, using the Klenow polymerase. The experiment in which this pre-pro fragment (174 pb) was clones and synthesised is shown on Figure 7.

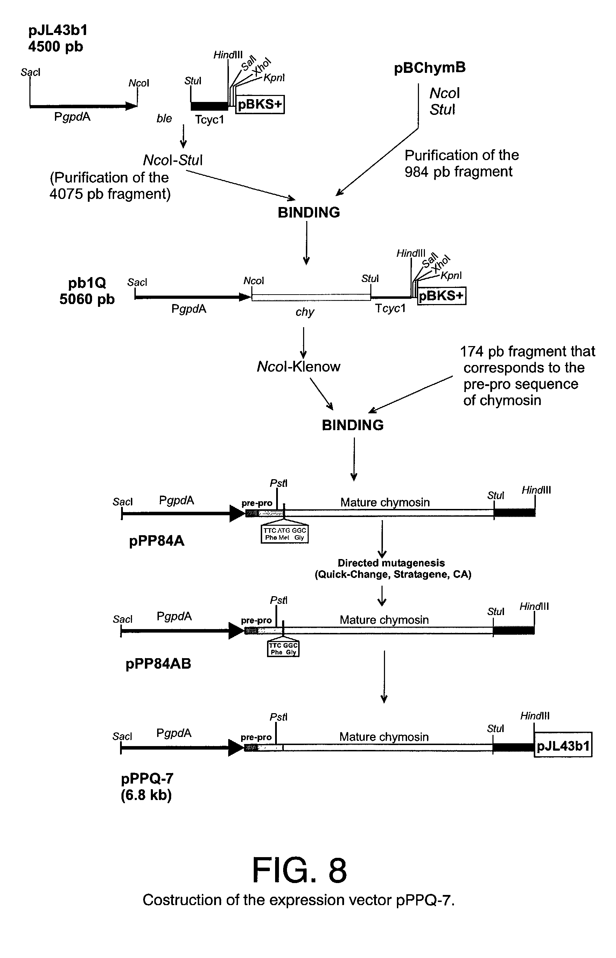

As can be seen on Figure 7, to sub-clone the 174 pb fragment that corresponds to the pre-pro sequence, both the pBChymB (Figure 6C) and the pb1Q (Figure 8) were digested with the *NcoI* enzyme and the ends were later completed with the Klenow fragment. The pre-pro fragment was later bound to this linearised plasmids giving rise to plasmids pPPQA and pPP84A, respectively (Figure 8). By an analysis of the sequence of 10 positive clones in each construction, we found that both the synthesis and the sub-cloning had been correctly performed. A mutagenesis experiment was later conducted in order to eliminate a TAG between the 3' end of the pre-pro fragment and the start of mature chymosin (Figure 8). From the plasmid pPP84AB (from pb1Q), it was digested with *Ec136II* and *HindIII* enzymes and sub-cloned in the pJL43bl, giving rise to the plasmid pPPQ-7 (Figure 8), which has been used to transform the 1pr66 stock of A. awamori.

### EXAMPLES

### 1.- Overexpression of the artificial chy gene in the filamentous fungus A. awamori.

There is a large bibliography related to the production of heterologous proteins in filamentous fungi (see the review by Gouka et al., 1997). It has been seen that, in general, the production of the protein of interest is low, compared with the production of homologous proteins from the productive micro-organism and that the production levels do not exceed a few milligrams per litre of culture media. Because of these limitations, different strategies have been developed to improve this production, which are mentioned as follows:
i).- Use of efficient expression signals: strong homologous promoters and efficient secretion signals.
ii).- Use of stock deficient in proteolithic activity.
iii).- Development of an adequate culture medium.
iv).- If necessary, modification of the protein (in this case, we used a synthetic gene, which, as was mentioned in the first section, was designed based on the use of Aspergillus niger codons).

### 2.- Promoter of the gpdA gene of Aspergillus nidulans.

The gpdA gene that encodes for the enzyme glyceraldehyde 3-phosphate dehydrogenase (GPD) of Aspergillus nidulans was isolated and sequenced by Punt et al (1988). The GPD protein is a key enzyme in glycolysis and in gluconeogenesis. It is also known that in this micro-organism, in Saccharomyces cerevisiae and in superior eucaryotes, it constitutes approximately 5% of the soluble cellular protein, which supports the conclusion that this gene's expression signals are very strong. The gpdA gene of Aspergillus nidulans is cloned in the plasmid pAN52.1 (Punt et al., 1988). From this plasmid it has been possible to sub-clone a 880 pb fragment of *SacI-NcoI,* which corresponds to a region of the promoter of the gpdA gene (Gutiérrez et al., 1997). This promoter has been used for the overexpression of different proteins in Aspergillus awamori, such as for example in the case of Thaumatin (Moralejo et al., 1999). The results obtained in the previously mentioned work have shown that this promoter also works efficiently in this micro-organism, so it has been used for the overexpression of chymosin.

### 3.- Transformation of the 1pr66 stock of A. awamori with the expression vector pPPQ-7.

The 1pr66 stock of A. awamori, which lacks the protease aspergillopepsin A, was transformed with the expression vector pPPQ-7. To obtain the different transformants, the protocol described by Yelton et al (1984) was followed, with slight modifications.

From a culture grown on a PW solid media dish (Mix 1:1 v:v of the media PM1 and Czapek-KC1) (Media PM1: Bacto peptone, 5 g; corn maceration solids, 1 g; lactose, 5 g; NaCl, 4 g; CuSO₄.5H₂O, 0.001 g; FeCl₃.6H₂O, 0.003 g; MgSO₄. 7H₂O, 0.05 g; KH₂PO₄, 0.06 g; Agar 2%) (Media Czapek-KCl: Saccharose, 30 g; NaNO₃, 2 g; K₂HPO₄, 0.5 g; MgSO₄.7H₂O, 0.5 g; FeSO₄.7H₂O, 0.01 g; KCl 0.7 M; Agar 2%), the spores were scraped and inoculated in a CM liquid media (malt extract, 5g; yeast extract, 5 g; glucose, 5 g). This culture was incubated for 12-14 h at 28°C and 300 rpm. The mycelium thus obtained was filtered through 30 µm pore diameter nytal tissue, washed with 0.9% NaCl once, and once again with the TLA wash buffer (sodium phosphate 0.01 M, magnesium sulphate 0.6 M, pH 5.8). 1 g of mycelium was re-suspended in 20 ml of TPA protoplast buffer (sodium phosphate 0.01 M, magnesium sulphate 1.2 M, pH 5.8), containing 3 mg/ml of Lysing (Sigma) lythic enzyme. It was incubated for 3-4 h at 301C and 100 rpm. The protoplasts were then recovered through nytal and then precipitated at 8,000 rpm for 3 min. The protoplasts obtained were washed twice with Tris-HCl 10 mM pH 7.5-Sorbitol 1 M (ST) and once with the same buffer but containing CaCl₂ 10 mM (STC). The protoplasts were finally re-suspended in a sufficient volume of STC to give a final concentration of 10⁸ protoplasts/ml, to which was added 1/10 volume of polyethylen glycol 6000 at 60% in a Tris-HCl 10 mM pH 7.5-CaCl₂ 10 mM (PTC) buffer. To 200 µl of protoplasts were added 20 µg of DNA and they were incubated in ice for 20 min. 1 ml of PTC was then added and they were incubated for another 20 min at room temperature. Finally, to the transformation mix were added 1.2 ml of STC and it was distributed equally in different tubes in order to distribute them as coverage on dishes containing the TSA-Sorbitol 1 M (Difco) media, with phleomycin at a concentration of 50 µg/ml. 40 transformants were selected, and they were allocated the initials TPPQ-X.

Because of the promoter used in the construction to express the preprochymosin, the MDFA media (1.2% L-asparagin, 3.6% saccharose, 2.7% glucose, inorganic salts) was used to carry out the fermentations to analyse the different transformants. They were all previously grown in CM media at 30°C and 250 rpm, in order to obtain a pre-inoculum. It was later inoculated with 18% /v/v) in 100 ml of MDFA production media.

### 4.- Analysis of the transformants containing the pPPQ-7 expression vector.

For the analysis of the rennin production, we collected culture supernatants every 24 hours from each of the transformants, including the control A. awamori 1 pr66 stock. From each stock, two equal amounts of 2 ml were collected. One of them was kept at 4°C and the remaining 2 ml had their pH adjusted to 2.5 with HCl 1 N, maintaining them in this condition for 1 h at room temperature, to study the induction of the processing of the rennin to an acid pH. Later, the pH was increased to 5.0-5.5 with NaOH 1 N and the milk clotting test was run (Emtage et al., 1983), which is based on the selective clotting of milk by chymosin, both for the supernatants the pH of which had not been modified (control) and those for which it had not.

Test method: To each pit of a microtitle dish was added 175 µl of a 12% solution of milk in buffer phosphate 25 mM, CaCl₂ 20 mM, pH 6.3. Later, a known concentration of calf chymosin (500 ng) and 175 µl of the supernatants of the different transformants, making serial dilutions in each row. The dish was incubated for 1-2 h at 37°C and later placed upside down on filter paper so that in the areas where the milk had not clotted it was eliminated from the reaction mix. The test's detection threshold is 100-200 ng of chymosin.

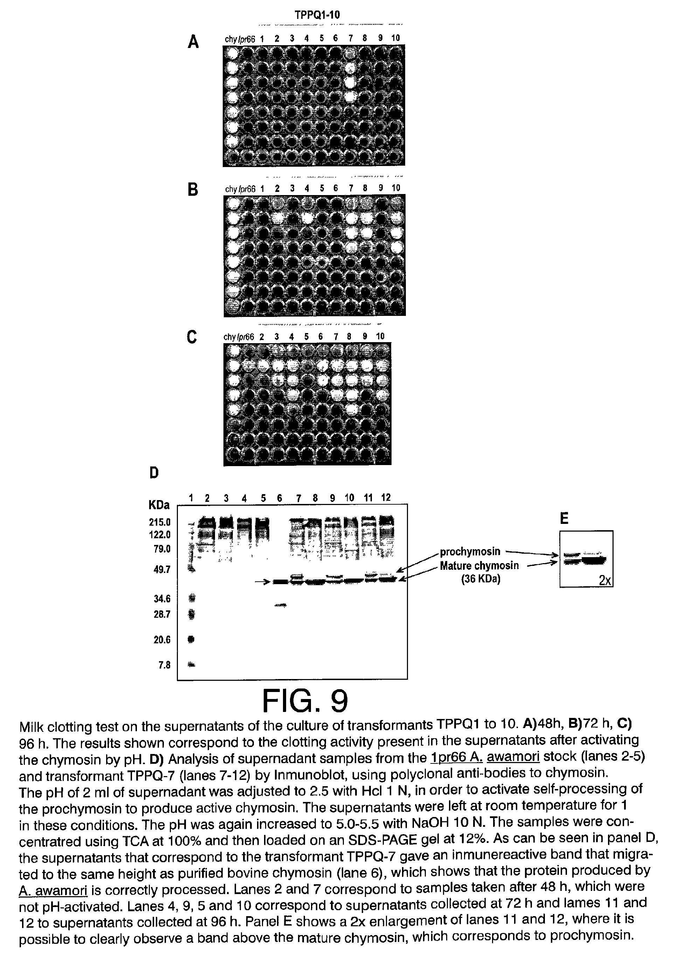

This parallel analysis was performed because during the growth of the different transformants in the MDFA media, the pH is maintained at values of 6.5-5.5 throughout fermentation, and these pH values are not adequate to activate the prochymosin to produce the active chymosin (Foltmann, 1979a). Figures 9A, 9B and 9C show the results obtained in the milk clotting test on the supernatants the pH of which was modified. After 48 h (Figure 9A), the supernadant of the TPPQ-7 transformant gave to clotting pits when its pH was not acidified (not shown in the figure). On the other hand, the supernadant from the same transformant, after altering the pH, gave at the end of the test 4 clotting pits. The same happened when the test was conducted after 72 h of growth, and it was also found that there were other transformants, such as TPPQ-8 and TPPQ-10, which also gave a milk clotting signal similar to that shown by TPPQ-7 (Figure 9B). These same results were repeated when the supernatants collected after 96 h of growth were collected (Figure 9C). The results indicated that it was possible that the prochymosin was secreting in the culture media and was processed, even in the alkaline pH conditions of the MDFA media, because it was possible to detect the clotting activity in the supernatants that were not pH-activated.

To confirm these results, the different supernatants from the 1pr66 stock of A. awamori and the TPPQ-7 stock were concentrated 10 times with TCA (10% final) and we applied to a polyacrylamide gel (DSD-PAGE) at 12% (Figures 9D and 9E). At the end of the electrophoresis, the gel proteins were transferred to a PVDF membrane (Immobilon from Millipore) and immune detection was performed using polyclonal anti-bodies to bovine chymosin. A figure 9D shows, the supernatants that corresponded to the A. awamori 1pr66 stock (lanes 2-5) gave a negative result compares to the signal obtained with purified bovine chymosin (lane 6). On the other hand, in the supernatants from the growth of TPPQ-7 transformant, it was possible to detect an immune-reactive band facing the anti-bodies used, which migrated at the same height as the purified bovine chymosin (lanes 7-12), which confirmed that the clotting activity detected in the supernatants from this stock corresponded to the chymosin secreted, which is also correctly processes. In all the samples of this transformant that were analysed, it was possible to detect another immune-reactive band that migrates above chymosin, which possibly corresponds to prochymosin (Figure 9E).

### BIBLIOGRAPHY

- Cullen D., Gray G.L., Wilson L.J., Hayenga K. J., Lamsa M.H., Rey M.W., Norton S. And Berka R.M. 1987. *Controlled expression and secretion of bovine chymosin in Aspergillus nidulans.* Bio/Technology, 5: 369-376.
- Dunn-Coleman N.S., Bloebaum P., Berka R.M., Bodie E., Robinson N., Armstrong G., Ward M., Petrak M., Carter G.L.,. LaCost R., Wilson L.J., Kodama K.H., Balin E.F., Bower B., Lamsa M. And Heinsohn H. 1991. *Commercial levels of chymosin production by Aspergillus.* Bio/Technology 9: 976-981.
- Emtage J.S., Angal S., Doel M.T., Harris T.J.R., Jenkins B., Lilley G. And Lowe P.A. 1983. *Synthesis of calf prochymosin (prorennin) in Escherichia coli.* Proc. Natl. Acad. Sci. 80: 3671-3675.
- Foltmann B., Pedersen V.B., Jacobsen H., Kauffman S. And Wybrant G. 1977. *The complete amino acid sequence of prochymosin.* Proc. Natl. Acad. Sci. 74: 2321-2324.
- Foltmann B. 1979a. *Prochymosin and chymosin (prorennin and rennin),* in Perlmann G.E. and Loranci L. (Eds), Methods in Enzymology, vol. 19. Academic Press, New York, pp 421-436.
- Foltmann B., Pedersen V.B., Kauffman D. And Wybrandt G. 1979b. *The primary structure of calf chymosin.* J. Biol. Chem. 254: 8447-8456.
- Gouka R.J., Punt P.J. and van den Hondel C.A.M.J.J. 1997. *Efficient production of secreted proteins by Aspergillus: progress, limitations and prospects.* Appl. Microbiol. Biotechnol. 47: 1-11.
- Gutiérrez S., Velasco J., Marcos A.T., Fernández F.J., Fierro F., Barredo J.L., Díez B and Martin J.F. 1997. *Expression of the cefG gene is limiting for cephalosporin biosynthesis in Acremonium chrysogenum.* Appl. Microbiol. Biotechnol. 48: 606-614.
- Hayden M.A. and Mandecki W. 1988. *Laboratory methods: Gene synthesis by serial cloning of oligonucleotides.* 7 (8): 571-577.
- Hill D.E., Hope I.A., Macke J.P. and Struhl K. 1986. *Saturation mutagenesis of the yeast His3 regulatory site: Requirements for transcriptional induction and binding by GCN4 protein.* Science. 234: 451-457-
- Matteucci M.D. and Heynecker H.L. 1983. *Targeted random mutagenesis: The use of ambiguously synthesised oligonucleotides to mutagenize sequences immediate 5' of an ATC initiation codon.* Nucl. Acids Res. 11: 3113-3121.
- Mellor J, Dobson M.J., Roberts N.A., Tuite M.F., Emtage J.S., White S., Lowe P.A., Patel T., Kingsman A.J. and Kingsman S.M. 1983. *Efficient synthesis of enzymatically active calf chymosin in Saccharomyces cerevisiae.* Gene. 24: 1-14.
- Moralejo F.J., Cardoza R.E., Gutiérrez S and Martin J.F. 1999. *Thaumatin production in Aspergillus awamori by use of expression cassettes with strong fungal promoters and high gene dosage.* Appl. Environ. Microbiol. 65: 1168-1174.
- Pedersen V.B., Christensen K.A. and Foltmann B. 1979. *Investigations on the activation of bovine prochymosin.* Eur. J. Biochem. 94: 573-580.
- Pitts J.E., Quinn D., Uusitalo J. And Penttila M. 1991. *Protein engineering of chymosin and expression in Trichoderma reesei.* Biochemical Society Transactions. 19.
- Punt P.J., Dingemanse M.A., Jacobs-Meijsing B.J.M., Pouwels P.H. and van den Hodçndel C.A.M.J.J. 1988. *Isolation and characterization of the glyceraldehyde* *hosphate dehydrogenase gene of Aspergillus nidulans.* Gene. 69: 49-57.
- Tsuchiya K., Gomi K., Kitamoto K., Kumagai Ch. And Tamura G. 1993. *Secretion of calf chymosin from the filamentous fungus Aspergillus orysae.* Appl. Microbiol. Biotechnol. 40: 327-332.
- Tsuchiya K., Nagashima T., Yamamoto Y., Gomi K., Kitamoto K, Kumagai C. And Tamura G. 1994. *High level secretion of calf chymosin using a glucoamylase-prochymosin fusion gene in Aspergillus orysae.* Biosc. Biotech. Bioche. 58: 895-899.
- Ward M., Wilson L.J., Kodama K.H., Rey M. And Berka R.M. 1990. *Improved production of chymosin in Aspergillus by expression as a glucoamylase-chymosin fusion.* Bio/Technology 8: 435-440.
- Yelton M.M., Hamer J.E. and Timberlake W.E. 1984. *Transformation of Aspergillus nidulans by using a trpC plasmid.* Proc. Natl. Acad. Sci. 81: 1470-1474. 
SEQ. ID No. 1

atg cgc tgc ctg gtc gtc ctg ctg gcc gtc ttc gcc ctg tcc cag ggc 48
Met Arg Cys Leu Val Val Leu Leu Ala Val Phe Ala Leu Ser Gln Gly
1               5                   10                  15
gcc gag atc acc cgc atc ccc ctg tac aag ggc aag tcc ctg cgc aag 96
Ala Glu Ile Thr Arg Ile Pro Leu Tyr Lys Gly Lys Ser Leu Arg Lys
            20                  25                  30
gcc ctg aag gag cac ggc ctg ctg gag gac ttc ctg cag aag cag cag 144
Ala Leu Lys Glu His Gly Leu Leu Glu Asp Phe Leu Gln Lys Gln Gln
        35                  40                  45
tac ggc atc tcc tcc aag tac tcc ggc ttc ggc gag gtc gcc tcc gtc 192
Tyr Gly Ile Ser Ser Lys Tyr Ser Gly Phe Gly Glu Val Ala Ser Val
    50                  55                  60
ccc ctg acc aac tac ctc gac agc cag tac ttc ggc aag atc tac ctg 240
Pro Leu Thr Asn Tyr Leu Asp Ser Gln Tyr Phe Gly Lys Ile Tyr Leu
65                  70                  75                  80
ggc acc ccc ccc cag gag ttc acc gtc ctc ttc gac acc ggc tcc agc 288
Gly Thr Pro Pro Gln Glu Phe Thr Val Leu Phe Asp Thr Gly Ser Ser
                85                  90                  95
gac ttc tgg gtc ccc tcc atc tac tgc aag agc aac gcc tgc aag aac 336
Asp Phe Trp Val Pro Ser Ile Tyr Cys Lys Ser Asn Ala Cys Lys Asn
            100                 105                 110
cac cag cgc ttc gac ccc cgc aag tcg tct acc ttc cag aac ctg ggt 384
His Gln Arg Phe Asp Pro Arg Lys Ser Ser Thr Phe Gln Asn Leu Gly
        115                 120                 125
aag cct ctc tct atc cac tac ggt acc ggt tct atg cag ggt atc ctt 432
Lys Pro Leu Ser Ile His Tyr Gly Thr Gly Ser Met Gln Gly Ile Leu
    130                 135                 140
gga tac gac act gtc act gtc tcc aac atc gtg gac atc cag cag acg 480
Gly Tyr Asp Thr Val Thr Val Ser Asn Ile Val Asp Ile Gln Gln Thr
145                 150                 155                 160
gtg ggc ctt agc acg cag gag cct ggc gat gtt ttc acc tac gcc gag 528
Val Gly Leu Ser Thr Gln Glu Pro Gly Asp Val Phe Thr Tyr Ala Glu
                165                 170                 175
ttc gat ggc atc ttg ggc atg gcc tat cct tcc ttg gcc agc gag tat 576
Phe Asp Gly Ile Leu Gly Met Ala Tyr Pro Ser Leu Ala Ser Glu Tyr
            180                 185                 190
tcc att ccg gtt ttt gat aac atg atg aac cgc cac ctg gtc gcc caa 624
Ser Ile Pro Val Phe Asp Asn Met Met Asn Arg His Leu Val Ala Gln
        195                 200                 205
gat ctc ttt agc gtc tat atg gac cgc aac ggc caa gag tcg atg ctg 672
Asp Leu Phe Ser Val Tyr Met Asp Arg Asn Gly Gln Glu Ser Met Leu
    210                 215                 220
acc ctc ggt gct att gac ccg tcg tac tac acc ggt tct ctg cac tgg 720
Thr Leu Gly Ala Ile Asp Pro Ser Tyr Tyr Thr Gly Ser Leu His Trp
225                 230                 235                 240
gtc ccc gtc acc gtc cag cag tac tgg cag ttc acc gtc gat tct gtg 768
Val Pro Val Thr Val Gln Gln Tyr Trp Gln Phe Thr Val Asp Ser Val
                245                 250                 255
acc att tcc ggt gtg gtt gtt gct tgc gag ggt gga tgc cag gct atc 816
Thr Ile Ser Gly Val Val Val Ala Cys Glu Gly Gly Cys Gln Ala Ile
            260                 265                 270
ctc gac act ggc act agc aag ctg gtc ggc ccc tcc agc gac atc ctt 864
Leu Asp Thr Gly Thr Ser Lys Leu Val Gly Pro Ser Ser Asp Ile Leu
        275                 280                 285
aat atc cag cag gcg atc ggc gcg acg cag aat cag tac ggc gaa ttc 912
Asn Ile Gln Gln Ala Ile Gly Ala Thr Gln Asn Gln Tyr Gly Glu Phe
    290                 295                 300
gac atc gat tgc gat aac ttg tcc tac atg ccc acg gtc gtc ttc gaa 960
Asp Ile Asp Cys Asp Asn Leu Ser Tyr Met Pro Thr Val Val Phe Glu
305                 310                 315                 320
atc aac ggc aag atg tac ccc ttg acc ccc agc gcc tac acc tcg caa 1008
Ile Asn Gly Lys Met Tyr Pro Leu Thr Pro Ser Ala Tyr Thr Ser Gln
                325                 330                 335
gat caa ggt ttc tgc acc tcg ggt ttc cag tct gaa aac cac tct cag 1056
Asp Gln Gly Phe Cys Thr Ser Gly Phe Gln Ser Glu Asn His Ser Gln
            340                 345                 350
aag tgg att ctg ggt gat gtc ttc att cgt gag tat tat tcc gtc ttc 1104
Lys Trp Ile Leu Gly Asp Val Phe Ile Arg Glu Tyr Tyr Ser Val Phe
        355                 360                 365
gac cgc gcc aac aac ctc gtc ggt ctg gcc aag gcc att tga 1146
Asp Arg Ala Asn Asn Leu Val Gly Leu Ala Lys Ala Ile .
370 375 380
ID sequence number 1: Sequence of chymosin amino-acids and sequence of the nucleotides of the artificial, synthetic and optimised gene, used in the example in this invention, to which the TAG end triplet has been added.

## Claims

1. A DNA sequence that encodes the sequence of amino acids that corresponds to the 382 amino acids of the chymosin protein (included in ID Sequence number 1), followed by a stop sequence; this DNA sequence is **characterised in that** it is the result of altering 100% of the DNA sequence of the natural gene that encodes the 382 amino acids of bovine chymosin (natural gene); this modification consists of the addition of a stop triplet of the TAG type, and in making 100% of the possible changes in the nucleotide triplets that correspond to the amino acids of the chymosin; these changes consists of the replacement of the original triplets by the triplets identified in brackets on the following list of amino acid triplets:

Ala (GCC), Arg (CGC), Asn (AAC), Asp (GAC), Cys (TGC), Gln
(CAG), Glu (GAG), Gly (GGC), His (CAC), Ilu (ATC), Leu
(CTG), Lys (AAG), Met (ATG), Phe (TTC), Pro (CCC), Ser
(TCC), Thr (ACC), Trp (TGG), Tyr (TAC), Val (GTC).

2. A DNA sequence, in accordance with claim 1, **characterised in that** all (100%) the possible triplet changes are made, so that said DNA sequence is as shown on ID Sequence number 1.

3. A recombinant plasmid **characterised in that** it includes: (i) a DNA sequence in accordance with either of claims 1 and 2, (ii) an expression cassette for filamentous fungi that contains a promoter sequence and an adequate end sequence for this type of fungi, (iii) an excretion signal so that the protein is produced extra-cellularly, and (iv) an adequate selection marker.

4. A recombinant plasmid in accordance with claim 3, **characterised in that** the sequence that promotes the expression cassette comes from the enzyme gene glyceraldehyde 3-phosphate deshydrogenase of Aspergillus nidulans; the sequence that ends the expression cassette is the cytochrome 1-oxydase of Sacharomyces cerevisiae; and the selection marker is resistance to phleomycin.

5. A recombinant plasmid that expresses the chymosin protein, **characterised in that** it includes: (i) an adequate selection marker; (ii) a DNA sequence made up of the DNA sequence in claim 1 which includes (a) a "pre" signal sequence and the "pro" sequence of the chy gene (chymosin), (b) a sequence that encodes for mature chymosin.

6. A filamentous fungus culture capable of producing the chymosin protein, which has been transformed in any of the plasmids in claims 3 to 5.

7. A culture in accordance with claim 6 where the filamentous fungus used is Aspergillus awamori.

8. A procedure for producing chymosin that consists of the following steps:
a).- incorporation of the DNA sequence in claim 1 in an expression vector selected from those that correspond to any of claims 3 to 5, by common recombinant DNA technology techniques;
b).- transformation in a filamentous fungus stock with the previous expression vector;
c). - culture of the filamentous fungus stock thus transformed, in culture media with the appropriate nutrients, producing chymosin extra-cellularly.
d).- separation and purification of the chymosin from other components of the culture media.

9. A procedure in accordance with claim 8, where the filamentous fungus selected in Aspergillus awamori.
